Europäisches Patentamt

European Patent Office

Office européen des brevets

(19)

(11) Publication number: **0 223 685**
A2

(12) # EUROPEAN PATENT APPLICATION

(21) Application number: **86402432.8**

(22) Date of filing: **30.10.86**

(51) Int. Cl.⁴: **C 12 Q 1/04**

(30) Priority: **31.10.85 JP 242971/85**

(43) Date of publication of application: **27.05.87**
**Bulletin 87/22**

(84) Designated Contracting States: **BE DE FR GB IT NL SE**

(71) Applicant: **TERUMO CORPORATION, 44-1, 2-chome, Hatagaya Shibuya-Ku, Tokyo 151 (JP)**

(72) Inventor: **Shigematsu, Takashi, 2-3-4-202, Nagayama, Tama-shi Tokyo (JP)**

(74) Representative: **Descourtieux, Philippe et al, CABINET BEAU de LOMENIE 55 rue d'Amsterdam, F-75008 Paris (FR)**

(54) **Medium composition for testing the microorganism capability of decarboxylating amino acids.**

(57) A medium composition for the identification of microorganisms is disclosed. The medium is used for testing the capability of decarboxylating amino acids of the glucose-nonfermentative Gram-negative bacilli. The medium has a controlled composition which comprises specified proportions of an amino acid, peptone, yeast extract, alkali metal dihydrogenphosphate, di-alkali metal hydrogenphosphate, glucose and a pH indicator and has a pH of 5.5-6.5 when dissolved in water. Use of the medium enables correct identification of the microorganisms independent of the cultivation period of time. Therefore, the identification may be made either on the same day or on the next day for the convenience of test operation.

EP 0 223 685 A2

Title of the Invention

Medium composition for testing the microorganism

capability of decarboxylating amino acids


Background of the Invention

1.  Field of Invention

This invention relates to a medium composition for testing the capability of microorganisms to decarboxylate amino acids.

More particularly, it is concerned with a medium composition for glucose-nonfermentative Gram-negative bacilli to test their capability of decarboxylating amino acids.

In order to apply an appropriate therapy for microbial infections it is important to identify pathogenic bacteria, conduct sensitivity tests and determine a drug active against the pathogenic bacteria.  In the identification of pathogenic bacteria for the above purpose, a variety of examinations of biochemical properties is carried out, among which is the aforementioned test for the capability of decarboxylating amino acids.  The medium composition of the invention is preferably utilized for such a test.


2.  Description of Prior Art

As the medium for the above-mentioned test has

heretofore been employed Møller medium having the

following composition:

(Møller medium)

| Amino acids | 10 (g) |
| Peptone | 5 |
| Meat extract | 5 |
| Pyridoxal | 0.005 |
| Glucose | 0.5 |
| Bromcresol Purple | 0.01 |
| Cresol Red | 0.005 |
| Distilled water | 1000 (ml) |
| pH | 6.0 |

Test for determining the microorganism capability of decarboxylating amino acids employing the above-mentioned prior-art medium requires a cultivation period of one to three days until a correct result of the test is found. On the other hand, in order to treat microbial infections at earlier stages it is necessary to carry out the identification of microorganisms as soon as possible, and development of the medium which enables identification of microorganisms in a shorter period of time has been desired. In some cases, however, the judgment is made inevitably on the next day for the convenience of operation. In such a case, it is desirable to employ a medium with which strict fixation of cultivation period of time is not required, and extension of the cultivation period of time by certain reasons does not cause an excess reaction.

## Summary of the Invention

It is an object of the present invention to provide a medium composition for glucose-nonfermentative Gram-negative bacilli to test their capability of decarboxylating amino acids which enables the identification by cultivation for a shorter period of time as well as makes it possible to identify the microorganism correctly after cultivation for a longer period of time.

A further object of the invention is to provide a medium composition for the above described test with which the color reaction is clear, and the judgment is easy.

According to the present invention, there is provided a medium composition for glucose-nonfermentative Gram-negative bacilli to test their capability of decarboxylating amino acids comprising 20 parts (by weight, the same hereinbelow) of amino acid, 0.15-1.5 (preferably 0.2-1.0) part of peptone, 0.008-0.8 (preferably 0.01-0.6) part of yeast extract, 0.05-1.0 (preferably 0.1-0.8) part of alkali metal dihydrogenphosphate, 0.05-0.8 (preferably 0.1-0.5) part of di-alkali metal hydrogenphosphate, 0.8-3.5 (preferably 1.0-3.0) parts of glucose and 0.01-0.2 (preferably 0.02-0.1) part of a pH indicator.

## Detailed Description of the Invention

As set forth above, the medium of the invention comprises an amino acid, peptone, a yeast extract, an alkali metal dihydrogenphosphate, a di-alkali metal hydrogenphosphate, glucose and a pH indicator. The amino acid is the substrate, for which any amino acid may be employed as desired, for example, L-lysine, L-arginine, L-ornithine or the like. It is preferred to employ the amino acid in the form of an acid addition salt such as hydrochloride, sulfate, nitrate or phosphate if it is a basic amino acid, and in the form of an alkali metal salt if it is an acidic amino acid. Peptone is a proteinous nutrient. As it is employed in one fifth or less than that employed in a known medium, test error (pseudo-positivity) caused by basic degradates such as ammonia can be avoided. Glucose is also used as a nutrient. As liquid paraffin cannot produce perfectly anaerobic conditions, glucose forms acidic degradates. Accordingly, the amount of glucose used is fixed in such a range as producing no test error. Yeast extract is included for promoting enzyme reactions and is used in such an amount as producing a pseudo-positive response. The alkali metal dihydrogenphosphate and di-alkali metal hydrogenphosphate act as pH-adjusting agents. As the alkali metal is used potassium or sodium. Combination of them is preferable. Suitable pH indicators are Phenol Red, Bromthymol Blue, Bromcresol Purple, Cresol Red, Bromphenol Red and the like, though any of pH indicators may be employed. Use of Phenol Red is especially suitable, because the color is clear and judgment whether it is positive or negative is easily made using a small amount of the examined material.

In addition to the above-mentioned components, it is preferred to use 5'-pyridoxalphosphate in order to promote the amino acid-decarboxylation reaction and an alkali metal chloride such as, for example, sodium chloride or potassium chloride as an osmotic pressure-adjusting agent.

The above-mentioned proportions of the components in the medium of the invention are critical. The pH-adjusting agent is fixed in such a way that pH is from 5.5 to 6.5 and preferably from 5.8 to 6.2 and the medium after the amino acid substrate has been decarboxylated has a pH of 7 or higher. Amount of the amino acid and the 5'-pyridoxalphosphate are also fixed in such a way that the cultivation time can be shortened.

Amount of the pH indicator is somewhat variable depending upon its nature. For example, Phenol Red is employed preferably in an amount form 0.04 to 0.1 part.

The culture medium composition of the invention is employed in liquid medium which is prepared by dissolving the components in predetermined amounts as defined above in water. When simultaneous examinations for a number of tests using a multiwell plate are to be carried out, as are usual in the biochemical test, a medium composition according to the invention is poured into a well in a test plate and dried to prepare a dry medium. In carrying out the test, an aqueous suspension of the microorganism to be tested in a predetermined amount is poured into each of the well prepared as above, cultivation is carried out at 30°C for a predetermined period of time. During the cultivation, the medium is layered with liquid paraffin.

The amino acid is converted by the decarboxylation reaction with the microorganism to a basic substance. For example, lysine is converted to cadaverine, arginine to agmatine, and ornithine to putrescine. The reaction is judged as positive or negative based upon pH change caused by production of such basic substance. If the judgment is to be made after a 4- to 5-hour cultivation, the medium is inoculated with $7.5 \times 10^7 - 1.05 \times 10^8$ organisms per 50 $\mu$l of the medium as calculated as a liquid medium. If the judgment is made after a 18- to 22-hour cultivation, it is preferred to inoculate the medium with $1.5 \times 10^7 - 3 \times 10^7$ organisms.

Examples are given below for the amino acid-decarboxylation test using the medium composition of the invention.

Examples

A medium composition of the invention with a composition as described in Tables 1-3 was dissolved in 1 lit. of water to prepare the media 1-9 for the identification of microorganisms.

Table 1

L-Lysine medium

| Component | Medium 1 | Medium 2 | Medium 3 |
|---|---|---|---|
| Peptone | 0.2 | 0.6 | 1.0 |
| Yeast extract | 0.1 | 0.4 | 0.6 |
| NaCl | 3 | 3 | 3 |
| 5'-Pyridoxalphosphate | 0.05 | 0.05 | 0.05 |
| $KH_2PO_4$ | 0.2 | 0.6 | 0.8 |
| $Na_2HPO_4$ | 0.1 | 0.3 | 0.5 |
| Glucose | 1 | 2 | 3 |
| L-Lysine hydrochloride | 20 | 20 | 20 |
| Phenol Red | 0.04 | 0.04 | 0.04 |
| Distilled water | 1000 | 1000 | 1000 |

(g)

Table 2

L-Arginine medium

| Component | Medium 4 | Medium 5 | Medium 6 |
|---|---|---|---|
| Peptone | 0.2 | 0.6 | 1.0 |
| Yeast extract | 0.1 | 0.4 | 0.6 |
| NaCl | 3 | 3 | 3 |
| 5'-Pyridoxalphosphate | 0.05 | 0.05 | 0.05 |
| $KH_2PO_4$ | 0.3 | 0.6 | 0.8 |
| $Na_2HPO_4$ | 0.1 | 0.2 | 0.4 |
| Glucose | 1 | 2 | 3 |
| L-Arginine hydrochloride | 20 | 20 | 20 |
| Phenol Red | 0.04 | 0.04 | 0.04 |
| Distilled water | 1000 | 1000 | 1000 |

(g)

Table 3

L-Ornithine medium

| Component | Medium 7 | Medium 8 | Medium 9 |
|---|---|---|---|
| Peptone | 0.2 | 0.6 | 1.0 |
| Yeast extract | 0.1 | 0.4 | 0.6 |
| NaCl | 3 | 3 | 3 |
| 5'-Pyridoxalphosphate | 0.05 | 0.05 | 0.05 |
| $KH_2PO_4$ | 0.1 | 0.2 | 0.4 |
| $Na_2HPO_4$ | 0.1 | 0.2 | 0.4 |
| Glucose | 1 | 2 | 3 |
| L-Ornithine hydrochloride | 20 | 20 | 20 |
| Phenol Red | 0.04 | 0.04 | 0.04 |
| Distilled water | 1000 | 1000 | 1000 |

(g)

The above-mentioned media 1-9 were poured respectively in an amount of 50 µl into a well of a multiwell plate and dried. A culture of various microorganisms on an agar plate, obtained by cultivation at 30°C for 18-24 hours, was suspended in 1.0 ml of sterilized distilled water to $1.5 \times 10^9$ microorganism per ml for the 4-hour run and $3 \times 10^8$ microorganisms per ml for the 20-hour run. Each well was then stab-inoculated with 50 µl of the suspension and covered with liquid paraffin. The plate was incubated at 30°C for a predetermined period of time. A cultivation using Møller medium was carried out for comparison. Positive or negative reaction for the amino acid-decarboxylation was judged by change in color
of the medium.

Results are shown in Tables 4-6.

## Table 4

### L-Lysine medium

| Medium Cultivation time (hr) Microorganism | Medium 1 | | Medium 2 | | Medium 3 | | Control medium | | |
|---|---|---|---|---|---|---|---|---|---|
| | 4 | 20 | 4 | 20 | 4 | 20 | 4 | 24 | 72 |
| Xanthomonas maltophilia ATCC 13637 | + | + | + | + | + | + | − | + | + |
| Pseudomonas cepacia ATCC 27515 | + | + | + | + | + | + | − | − | + |
| Pseudomonas aeruginosa ATCC 10145 | − | − | − | − | − | − | − | − | − |
| Pseudomonas putida ATCC 12633 | − | − | − | − | − | − | − | − | − |

## Table 5

### L-Arginine medium

| Medium Cultivation time (hr) Microorganism | Medium 4 | | Medium 5 | | Medium 6 | | Control medium | | |
|---|---|---|---|---|---|---|---|---|---|
| | 4 | 20 | 4 | 20 | 4 | 20 | 2.4 | 48 | 72 |
| Pseudomonas putida ATCC 12635 | + | + | + | + | + | + | − | + | + |
| Pseudomonas aeruginosa ATCC 10145 | + | + | + | + | + | + | + | + | + |
| Pseudomonas fluorescens ATCC 13528 | + | + | + | + | + | + | − | + | + |
| Pseudomonas stutzeri ATCC 17558 | − | − | − | − | − | − | − | − | − |
| Pseudomonas cepacia ATCC 27515 | − | − | − | − | − | − | − | − | − |

Table 6

L-Ornithine medium

| Medium / Cultivation time (hr) / Microorganism | Medium 7 | | Medium 8 | | Medium 9 | | Control medium | | |
|---|---|---|---|---|---|---|---|---|---|
| | 4 | 20 | 4 | 20 | 4 | 20 | 4.8 | 72 | 96 |
| Pseudomonas cepacia ATCC 27515 | + | + | + | + | + | + | – | – | + |
| Alteromonas putrefaciens ATCC 4720 | + | + | + | + | + | + | + | + | + |
| Pseudomonas aeruginosa ATCC 10145 | – | – | – | – | – | – | – | – | – |
| Pseudomonas putida ATCC 12633 | – | – | – | – | – | – | – | – | – |

As apparent from Tables 4-6, use of the medium of the present invention in the test for the capacity of decarboxylating amino acids of glucose-nonfermentative Gram-negative bacilli enables correct identification of the microorganism independent of the cultivation time. Therefore, the identification may be made either on the same day or on the next day for the convenience of examination operation. On the contrary, use of the control medium needs cultivation for at shortest 24 hours thereby making the identification on the same day infeasible. With the control medium, for example, 24-hour cultivation of Pseudomonas cepacia ATCC 27515, Pseudomonas putida ATCC 12635 or Pseudomonas fluorescens ATCC 13528 gives negative reaction, and after the 48- to 96-hour

0223685

cultivation the reaction becomes positive. On the contrary, use of the medium of the invention enables correct judgment by the 4-hour cultivation with any of the microorganisms.

What is claimed is:

1.     Medium composition for testing the capability of decarboxylating amino acids of the glucose-nonfermentative Gram-negative bacilli comprising 20 parts (by weight, the same below) of an amino acid, 0.15-1.5 part of peptone, 0.008-0.8 part of yeast extract, 0.05-1.0 part of alkali metal dihydrogenphosphate, 0.05-0.8 part of di-alkali metal hydrogenphosphate, 0.8-3.5 parts of glucose and 0.01-0.2 part of a pH indicator.

2.     Medium composition according to Claim 1 which comprises 20 parts (by weight, the same below) of an amino acid, 0.2-1.0 part of peptone, 0.01-0.6 part of yeast extract, 0.1-0.8 part of alkali metal dihydrogenphosphate, 0.1-0.5 part of di-alkali metal hydrogenphosphate, 1.0-3.0 parts of glucose and 0.02-0.1 part of a pH indicator.

3.     Medium composition according to Claim 1 or 2 wherein the alkali metal dihydrogenphosphate is potassium dihydrogenphosphate and the di-alkali metal hydrogenphosphate is disodium hydrogenphosphate.

4.     Medium composition according to Claims 1 to 3 wherein the amino acid is L-lysine, L-arginine or L-ornithine.

5.      Medium composition according to Claims 1 to 4 wherein the pH indicator is Phenol Red, Bromcresol Purple or Cresol Red.